# EUROPEAN PATENT APPLICATION

(11) **EP 2 130 901 A1**
(43) Date of publication of application: **09.12.2009**
(21) Application number: 08721686.7
(22) Date of filing: 10.03.2008
(51) Int. Cl.: C12M 1/00

(54) **CULTURE APPARATUS**

(30) Priority: 12.03.2007 JP 2007061628
(71) Applicant: Sanyo Electric Co., Ltd., Osaka 570-8677 (JP)
(72) Inventor: BUSUJIMA, Hiroki, Ota-shi Gunma 373-0021 (JP); NAKAO, Atsushi, Neyagawa-shi Osaka 572-0021 (JP)
(74) Representative: Grey, Ian Michael
(86) International application number: PCT/JP2008/054270
(87) International publication number: WO 2008/111548

(57) **Abstract**

An incubation apparatus is provided to incubate culture targets such as cells, embryos or microorganisms in an incubation chamber. The incubation apparatus is capable of inexpensively, highly accurately and automatically calibrating a gas concentration sensor. The incubation apparatus includes a gas concentration sensor 42 which detects a gas concentration in the incubation chamber 4, control means (controller 70) for controlling the supply of a gas into the incubation chamber on the basis of an output of the gas concentration sensor 42, and standard gas supply means for supplying the gas concentration sensor 42 with a standard gas which has been previously adjusted to an actually used gas concentration or a gas concentration close thereto. The control means (controller 70) maintains the concentration of the standard gas, supplies the standard gas to the gas concentration sensor 42 by the standard gas supply means, and on the basis of an output of the gas concentration sensor 42 and the concentration of the standard gas at the moment, calibrates the actually used gas concentration of the gas concentration sensor 42.

## Description

### BACKGROUND OF THE INVENTION

The present invention relates to an incubation apparatus for incubating culture targets such as cells, embryos or microorganisms in an incubation chamber.

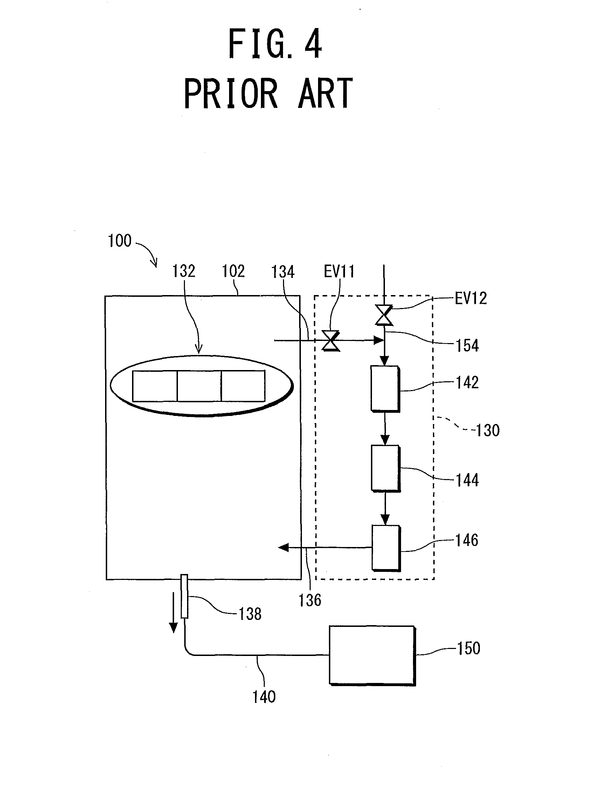

In a conventional incubation apparatus called an incubator, a constant temperature in an incubation chamber and a constant concentration of a gas such as a carbon dioxide gas (CO₂) or an oxygen gas (O₂) are maintained, and an aseptic condition is kept in the incubation chamber, in order to incubate culture targets (specimens) such as cells, embryos or microorganisms as incubation targets. As shown in FIG. 4, an incubation apparatus 100 includes an adiabatic box main body 102. The adiabatic box main body 102 is composed of a metal outer box which is open on one side, and a stainless-steel inner box provided inside the outer box. An incubation chamber is formed in this adiabatic box main body 102.

The inside of the incubation chamber is vertically partitioned by a plurality of shelves. Containers containing cultures are mounted on these shelves, and the cultures are put in and out of the incubation chamber by opening and closing a door which blocks the opening of the adiabatic box main body 102. A temperature suitable for incubation is maintained in the incubation chamber by a heater so that culture targets such as cells, embryos or microorganism are incubated. The incubation chamber has to maintain an environment suitable for the culture to be incubated, and therefore needs to keep a constant concentration of an incubation gas.

Accordingly, to measure the gas concentration in the incubation chamber, the adiabatic box main body 102 is provided with a measurement tube 138 which is blocked by an unshown rubber plug. Then, the rubber plug of the measurement tube 138 is removed, and a gas concentration measurement unit 150 prepared in advance is connected to the measurement tube 138 by an elastic hose 140. Further, a gas is taken from the incubation chamber into the gas concentration measurement unit 150 and measured, thereby checking the gas concentration and components in the incubation chamber (see Japanese Patent Application Laid-Open No. 2005-118021).

Meanwhile, a gas concentration sensor in a generally used gas concentration measurement device is subject to measurement errors due to the temperature, humidity or a change with time. Therefore, the gas concentration sensor is periodically calibrated. The incubation apparatus 100 is provided with a gas concentration measurement unit 130 which includes a gas concentration sensor 142 capable of measuring the concentration of a gas such as a carbon dioxide gas or an oxygen gas, an oxygen gas measurement sensor 144 and an air pump 146. This gas concentration measurement unit 130 is connected to the incubation apparatus 100 by pipes 134, 136 for guiding the gas in the incubation chamber.

An electromagnetic valve EV11 is provided in the middle of the pipe 134. This pipe 134 branches between the electromagnetic valve EV11 and the gas concentration sensor 142, where an outside air take-in pipe 154 having an electromagnetic valve EV12 is connected to the pipe 134.
In addition, 132 denotes a liquid crystal display device. This display device displays, for example, the temperature and humidity in the incubation chamber, the concentration of the carbon dioxide gas detected by the gas concentration sensor 142, and the amount of oxygen detected by the oxygen gas measurement sensor 144.

Furthermore, an operator powers the gas concentration measurement unit 130 on, closes the electromagnetic valve EV11 and opens the electromagnetic valve EV12 to drive the air pump 146. As a result, outside air is taken through the outside air take-in pipe 154, and the gas concentration in the outside air is measured by the gas concentration sensor 142, and then the concentration is displayed on the display device 132. In addition, the gas concentration in the air is substantially zero. Therefore, when the value of the measured concentration is not zero, the operator calibrates the gas concentration measurement unit 130 so that the display device 132 indicates zero.

After the calibration of the gas concentration measurement unit 130, the operator opens the electromagnetic valve EV11 and closes the electromagnetic valve EV12. As a result, the gas in the incubation chamber is taken into the gas concentration measurement unit 130 through the pipe 134, and the gas concentration in the incubation chamber is measured by the gas concentration sensor 142, and then the gas concentration is displayed on the display device 132. Thus, the gas concentration in the incubation chamber is correctly measured. Further, the gas concentration measurement unit 150 is calibrated at the correctly measured gas concentration in the incubation chamber. The gas concentration measurement unit 150 is calibrated periodically (roughly, once a day to once a week) .

On the other hand, there is one type of carbon dioxide gas incubation apparatus or oxygen gas/carbon dioxide gas incubation apparatus used for incubating cells, embryos or microorganisms, wherein a standard gas (e.g., carbon dioxide gas = 5%, oxygen gas = 5%, nitrogen gas (N₂) = 90%) adjusted to an actually used concentration in this incubation apparatus is continuously passed slowly into the incubation apparatus to achieve incubation. The advantage of this incubation apparatus is that there is no variation of the gas concentration and that an environment with a highly accurate gas concentration can be obtained. However, the price of the standard gas used for this incubation apparatus is extremely high.

Accordingly, there is another type of incubation apparatus designed to adjust the concentration of an inexpensive gas. This incubation apparatus is provided with a gas concentration sensor which connects a carbon dioxide gas, nitrogen gas or oxygen gas to the incubation apparatus and which measures the concentration of the gas in the incubation apparatus. Thus, an actually used concentration in this incubation apparatus is created. The incubation gas used by this incubation apparatus is lower in price than the incubation gas used by the former incubation apparatus. Moreover, this incubation apparatus can adjust the gas to any concentration. Therefore, the use of this incubation apparatus has been predominant in the current market.

### SUMMARY OF THE INVENTION

However, in the incubation apparatus described above, the gas concentration sensor has to be periodically calibrated at the actually used concentration of the incubation gas in the incubation chamber or at a concentration close thereto. Therefore, the incubation gas in the incubation chamber has to be brought to the actually used concentration or a concentration close thereto every measurement of the gas concentration in the incubation chamber, so that the measurement of the concentration of the incubation gas is disadvantageously complicated and troublesome.

The present invention has been made in view of the foregoing problem of the conventional technique, and is directed to provide an incubation apparatus for incubating culture targets such as cells, embryos or microorganisms in an incubation chamber, wherein a gas concentration sensor can be calibrated inexpensively, highly accurately and automatically.

That is, an incubation apparatus of the first invention incubates culture targets such as cells, embryos or microorganisms in an incubation chamber, and the incubation apparatus is characterized by including a gas concentration sensor which detects a gas concentration in the incubation chamber; control means for controlling the supply of a gas into the incubation chamber on the basis of an output of the gas concentration sensor; and standard gas supply means for supplying the gas concentration sensor with a standard gas which has been previously adjusted to an actually used gas concentration or a gas concentration close thereto, wherein the control means maintains the concentration of the standard gas, supplies the standard gas to the gas concentration sensor by the standard gas supply means, and on the basis of an output of the gas concentration sensor and the concentration of the standard gas at the moment, calibrates the actually used gas concentration of the gas concentration sensor.

The incubation apparatus of the second invention further includes outside air supply means for supplying outside air to the gas concentration sensor in the first invention, and the control means is characterized by supplying the outside air to the gas concentration sensor by the outside air supply means, and on the basis of an output of the gas concentration sensor at the moment, performing zero-point calibration of the gas concentration sensor.

The incubation apparatus of the third invention further includes a detachable cylinder in which the standard gas is hermetically contained, in the first or second invention, and the standard gas is supplied from the cylinder to the gas concentration sensor.

The incubation apparatus of the fourth invention further includes a connection opening to connect a pipe which supplies the standard gas to the gas concentration sensor, in any one of the first to third inventions.

The incubation apparatus of the fifth invention is caricaturized in that the control means calibrates the actually used gas concentration at preset time intervals, in any one of the first to fourth inventions.

According to the present invention, there is provided an incubation apparatus to incubate culture targets such as cells, embryos or microorganisms in an incubation chamber. This incubation apparatus includes: a gas concentration sensor which detects a gas concentration in the incubation chamber; control means for controlling the supply of a gas into the incubation chamber on the basis of an output of the gas concentration sensor; and standard gas supply means for supplying the gas concentration sensor with a standard gas which has been previously adjusted to an actually used gas concentration or a gas concentration close thereto, wherein the control means maintains the concentration of the standard gas, supplies the standard gas to the gas concentration sensor by the standard gas supply means, and on the basis of an output of the gas concentration sensor and the concentration of the standard gas at the moment, calibrates the actually used gas concentration of the gas concentration sensor. As a result, the gas concentration sensor can be automatically calibrated at the actually used concentration or a concentration close thereto.

Thus, the gas concentration sensor can be calibrated at the actually used gas concentration which is actually used in the incubation chamber, so that the gas concentration sensor can be calibrated suitably for actual use to achieve highly accurate incubation. Especially, there is no need to use, for example, a special gas concentration measurement instrument to measure the gas concentration in the incubation chamber, thereby enabling a significant improvement in maintenance work.

Furthermore, according to an aspect of the second invention, the incubation apparatus further includes outside air supply means for supplying outside air to the gas concentration sensor, and the control means supplies the outside air to the gas concentration sensor by the outside air supply means, and on the basis of an output of the gas concentration sensor at the moment, performs zero-point calibration of the gas concentration sensor. Thus, the gas concentration sensor can be calibrated at two points including a zero gas concentration and the actually used gas concentration, thereby enabling more accurate calibration.

Still further, according to an aspect of the third invention, the incubation apparatus further includes a detachable cylinder in which the standard gas is hermetically contained, and the standard gas is supplied from the cylinder to the gas concentration sensor. Thus, there is no need for a pipe for supplying the standard gas from an external gas supply source.

Further yet, according to an aspect of the fourth invention, the incubation apparatus further includes a connection opening to connect a pipe which supplies the standard gas to the gas concentration sensor. Thus, when the incubation apparatus is installed in a facility which is already equipped with a line to supply the standard gas, this standard gas line can be connected to the connection opening to supply the standard gas to the gas concentration sensor.

Further yet, according to an aspect of the fifth invention, the control means calibrates the actually used gas concentration at preset time intervals. This makes it possible to always achieve accurate incubation, and to hold down the amount of the use of the relatively expensive standard gas.

### BRIEF DESCRIPTION OF THE DRAWINGS

FIG. 1 is a longitudinal sectional side view showing the structure of an incubation apparatus in one embodiment of the present invention;
FIG. 2 is a diagram explaining the schematic structure of the incubation apparatus of the present invention;
FIG. 3 is a block diagram of a control circuit provided in the incubation apparatus of the present invention; and
FIG. 4 is a diagram explaining the schematic structure of a conventional incubation apparatus.

### DETAILED DESCRIPTION OF THE PREFERRED EMBODIMENT

The main object of the present invention is to inexpensively, highly accurately and automatically calibrate a gas concentration sensor for examining the concentration of an incubation gas. The object of inexpensively, highly accurately and automatically calibrate the gas concentration sensor is achieved by calibrating the actually used gas concentration of the gas concentration sensor on the basis of a standard gas previously adjusted to the actually used gas concentration. Embodiment 1

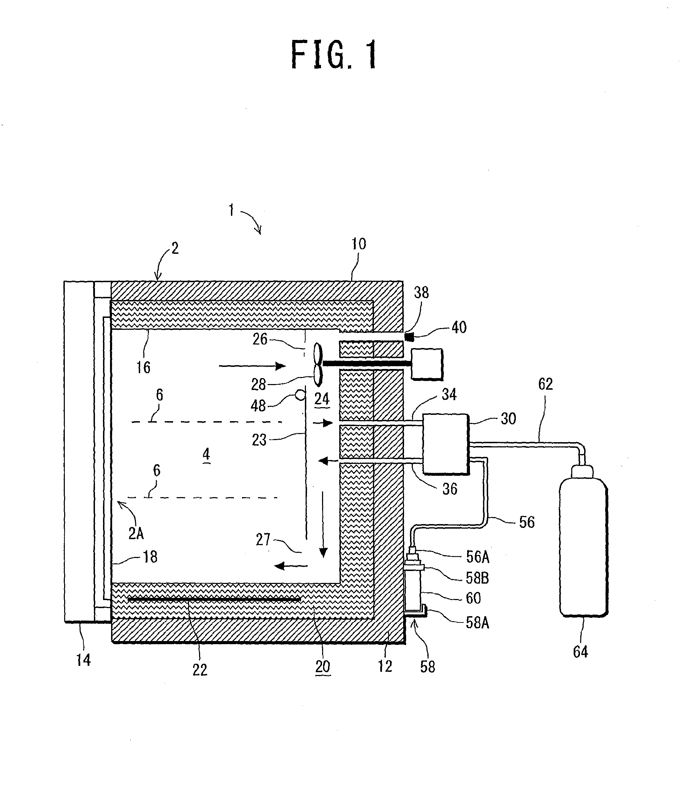

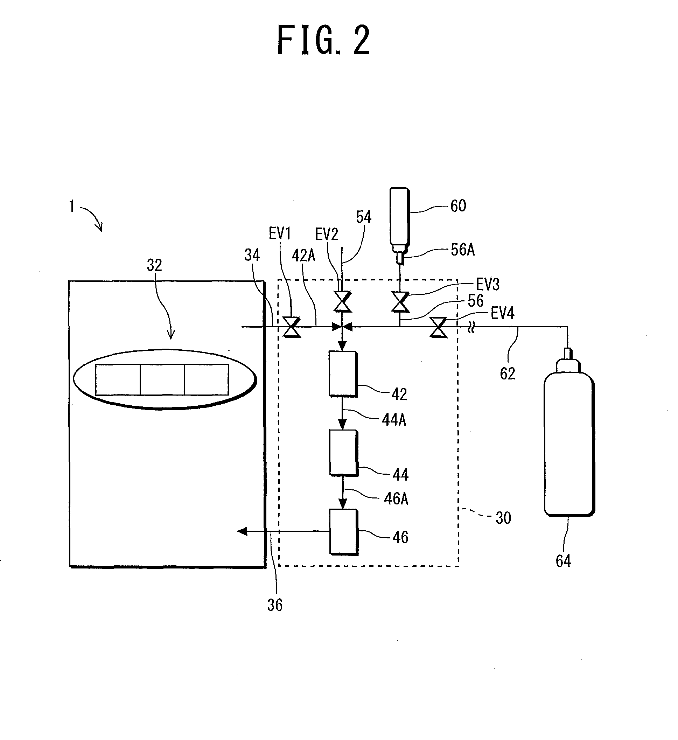

An embodiment of the present invention will be described below with reference to the drawings. FIG. 1 is a longitudinal sectional side view showing the structure of an incubation apparatus 1 representing one embodiment of the present invention. FIG. 2 is a diagram showing the schematic structure of the incubation apparatus 1 of the present invention.

As shown in FIG. 1, the incubation apparatus 1 of the present embodiment includes an adiabatic box main body 2. The adiabatic box main body 2 is composed of a metal outer box 10 in which an opening 2A is formed on one side, and a stainless-steel inner box 16 provided inside the outer box. The opening 2A of the inner box 16 is provided with a transparent inner door 18 and an outer door 14 outside the inner door 18. The right side of the inner door 18 is supported on the adiabatic box main body 2 by hinges so that the inner door is freely opened/closed. The right side of the outer door 14 is similarly supported by hinges so that the outer door is freely opened/closed. The inner door 18 airtightly blocks the opening 2A by a gasket (not shown) provided at the opening 2A of the adiabatic box main body 2.

Furthermore, an incubation chamber 4 is formed in a space (within the inner box 16) enclosed by the inner door 18 which blocks the opening 2A in a freely opened/closed manner. A plurality of shelves 6 (two shelves in the embodiment) are provided in the incubation chamber 4, and the inside of the incubation chamber 4 is vertically partitioned by the shelves 6. A culture of cells, embryos or microorganisms to be stored in the incubation chamber 4 is put in and out of the incubation chamber 4 by opening and closing the outer door 14 and the inner door 18. In addition, containers (not shown) containing the cultures are mounted on the shelves 6.

A heat insulating material 12 for heat retention is provided inside the outer box 10, and a circulation passage 20 for air or water is formed between the inner box 16 and the outer box 10. A heater 22 is disposed in the circulation passage 20 (the lower part of the incubation chamber 4). This heater 22 is heated so that the heat of the heater 22 is conducted to the inner box 16 and also conducted into the incubation chamber 4 by the air or water from the circulation passage 20, thereby keeping a predetermined temperature suitable for incubation in the incubation chamber 4.

A liquid crystal display device 32 (shown in FIG. 2) is provided in the front surface of the incubation apparatus 1, and a temperature sensor 48 is provided in the incubation chamber 4. For example, the temperature in the incubation chamber 4 measured by the temperature sensor 48 and the humidity detected by an unshown hygrometer are displayed on the display device 32. For example, the concentration of a carbon dioxide gas detected by a gas concentration sensor 42 described later and the amount of oxygen detected by an oxygen gas measurement sensor 44 are also displayed on the display device 32.

Furthermore, a back wall 23 is provided on the rear side of the incubation chamber 4, and a duct 24 is provided between the back wall 23 and the inner box 16. An inlet 26 in communication with the incubation chamber 4 is provided in the upper part of the duct 24, and an outlet 27 is provided in the lower part of the duct 24. Further, an air circulation fan 28 for controlling the environment in the incubation chamber 4 is provided in the duct 24, and this fan 28 is provided at a position corresponding to the inlet 26. Air in the incubation chamber 4 is sucked into the duct 24 from the inlet 26 by the fan 28, and the sucked air is blown into the incubation chamber 4 from the outlet 27 in the lower part of the duct 24 (arrow in FIG. 1). Thus, the air in the incubation chamber 4 is forced to circulate.

The incubation apparatus 1 is provided with a measurement hole 38 blocked with a rubber plug 40 to measure the gas concentration in the incubation chamber 4. This rubber plug 40 of the measurement hole 38 is attached and detached by an operator so that the gas concentration and components in the incubation chamber 4 can be checked.

The incubation apparatus 1 is further provided with a gas concentration measurement unit 30 for measuring the concentration of a carbon dioxide gas (CO₂) or an oxygen gas (O₂) supplied into the incubation chamber 4. As shown in FIG. 2, the gas concentration measurement unit 30 includes the gas concentration sensor 42 for measuring the gas concentration in the incubation chamber 4 (in the duct 24), the oxygen gas measurement sensor 44 for measuring the concentration of the oxygen gas, and an air pump 46 for circulating the air and gas through pipes. In addition, the technique for measuring the concentration of the carbon dioxide gas by the gas concentration sensor 42 and the technique for measuring the concentration of the oxygen gas by the oxygen gas measurement sensor 44 have heretofore been well known and are therefore not described in detail.

Two pipes 34, 36 in communication with the incubation chamber 4 are connected to the gas concentration measurement unit 30 to guide the gas in the incubation chamber 4 (in the duct 24) to the gas concentration sensor 42 and the oxygen gas measurement sensor 44. One pipe 34 extends out of the incubation chamber 4 into the gas concentration measurement unit 30, and is connected to the entrance of an electromagnetic valve EV1 which turns on/off the circulation of the incubation gas. The exit of the electromagnetic valve EV1 is connected to a pipe 42A at the entrance of the gas concentration sensor 42, and the exit of the gas concentration sensor 42 is connected to a pipe 44A at the entrance of the oxygen gas measurement sensor 44.

The exit of the oxygen gas measurement sensor 44 is connected to a pipe 46A at the entrance of the air pump 46, and the exit of the air pump 46 is connected to the pipe 36. That is, the two pipes 34, 36 are open in the incubation chamber 4, and one pipe 34 is in communication with the other pipe 36 via the electromagnetic valve EV1, the pipe 42A, the gas concentration sensor 42, the pipe 44A, the oxygen gas measurement sensor 44, the pipe 46A and the air pump 46. This pipe 34 is open in the incubation chamber 4.

The air pump 46 sucks the gas in the duct 24 (in the incubation chamber 4) from one pipe 34, and discharges the sucked gas into the duct 24 from the other pipe 36. Thus, the gas concentration measurement unit 30 can detect the gas concentration in the incubation chamber 4 by the gas concentration sensor 42 and the oxygen gas measurement sensor 44.

The pipe 42A branches between the electromagnetic valve EV1 and the gas concentration sensor 42, and is connected, via an electromagnetic valve EV2, to an outside air take-in pipe 54 for taking in the outside air into the gas concentration measurement unit 30. The end of this outside air take-in pipe 54 is open in the atmosphere. Moreover, the pipe 42A branches between the electromagnetic valve EV1 and the gas concentration sensor 42 so that a gas in-take pipe 56 is connected to the pipe 42A. A connector 56A (corresponding to a connection opening of the present invention) is provided at the end of the gas in-take pipe 56 via an electromagnetic valve EV3.

A cylinder 60 is connected to the connector 56A. This cylinder 60 is a so-called detachable cartridge type in which about 300 to 500 ml of a highly accurately adjusted standard gas (e.g., carbon dioxide gas = 5%, oxygen gas = 5%, nitrogen gas (N₂) = 90%) is hermetically contained. This standard gas is set at the actually used concentration (described in the example of the conventional technique) at which culture targets such as cells, embryos or microorganisms are actually incubated in the incubation apparatus 1. Standard gas supply means in the present embodiment is composed of the cylinder 60, the electromagnetic valve EV3 and a controller 70. Outside air supply means is composed of the electromagnetic valve EV2 and the outside air take-in pipe 54. In addition, the cylinder 60 is not limited to the above-mentioned capacity.

This cylinder 60 is detachably attached to the main body 2 of the incubation apparatus 1 by a holder 58. The holder 58 is composed of a holding table 58A on which the cylinder 60 is mounted, and a holding member 58B (FIG. 1). That is, the cylinder 60 is held on the holding table 58A fixed to the main body 2, and detachably attached to the main body 2 by the holding member 58B. Thus, when the standard gas hermetically contained in the cylinder 60 has run out, the cylinder 60 is replaced with another cylinder which is similar in configuration to the cylinder 60 and in which the standard gas is hermetically contained.

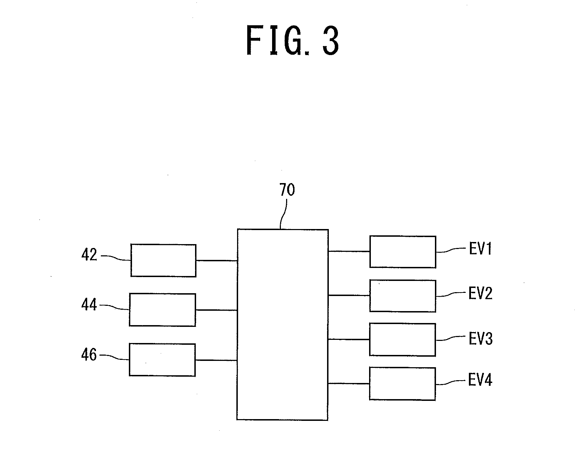

On the other hand, the gas concentration measurement unit 30 includes the controller 70 (corresponding to a controller 70 of the present invention) which is configured by a general-purpose microcomputer equipped with a memory and a timer and which controls the operation of the gas concentration measurement unit 30 (FIG. 3). The gas concentration sensor 42, the oxygen gas measurement sensor 44 and the air pump 46 are connected to the controller 70. The plurality of electromagnetic valves EV1, EV2, EV3, EV4 connected to the pipes of the above-mentioned sensors and air pump, and the display device 32 are also connected to the controller 70. In addition, the electromagnetic valve EV4 will be described later.

Here, what is especially important in the incubation apparatus 1 is the concentration of the carbon dioxide gas, so that the gas concentration in the incubation chamber 4 is measured, for example, every four hours. That is, in order to measure the gas concentration in the incubation chamber 4, the operator turns on a power switch (at this point, the electromagnetic valve EV1 is open, and the electromagnetic valves EV2, EV3, EV4 are closed). Then, the controller 70 operates the air pump 46 to suck the air in the incubation chamber 4 through the pipe 34 and discharge the air into the empty incubation chamber 4 through the pipe 36. That is, the incubation gas in the incubation chamber 4 (in the duct 24) repeats the circulation of flowing into the pipe 34 and returning into the incubation chamber 4 (the duct 24) from the pipe 36 through the gas concentration sensor 42 and the oxygen gas measurement sensor 44.

Furthermore, the concentration of the carbon dioxide gas in the incubation gas in the incubation chamber 4 when passing through the gas concentration sensor 42 is measured, and the concentration of the oxygen gas when passing through the oxygen gas measurement sensor 44 is measured. These concentrations are displayed on the display device 32. The standard concentrations of the carbon dioxide gas and the oxygen gas in the incubation chamber 4 are previously retained (stored) in the controller 70. The controller 70 compares the concentration of the carbon dioxide gas detected by the gas concentration sensor 42 with the concentration of the oxygen gas detected by the oxygen gas measurement sensor 44.

The controller 70 performs the comparison of the concentrations, for example, every four hours. When the concentration of the carbon dioxide gas or oxygen gas is insufficient, a predetermined amount of carbon dioxide gas or oxygen gas connected to the gas concentration measurement unit 30 is supplied to maintain the concentration of the carbon dioxide gas or oxygen gas in the incubation chamber 4 at the concentration of a set value.

Here, due to, for example, the temperature, humidity or a change with time, measurement errors are made in the gas concentration sensor 42 for measuring the gas concentration in the incubation apparatus and the oxygen gas measurement sensor 44. This error is displayed on the display device 32. Thus, the gas concentration sensor 42 needs to be calibrated. That is, in order to calibrate the gas concentration sensor 42, the electromagnetic valves EV1, EV3, EV4 are closed and the electromagnetic valve EV2 is opened by the controller 70 to operate the air pump 46, so that the outside air is taken in through the outside air take-in pipe 54.

Furthermore, the controller 70 stores the concentration of the carbon dioxide gas in the outside air taken in through the outside air take-in pipe 54 in a memory as zero. In this case, the concentration of the carbon dioxide gas in the outside air is substantially equal to zero, and the outside air serves as the reference of the concentration of the carbon dioxide gas.

Now, while some gas concentration sensors 42 can linearly detect the amount of carbon dioxide gas from 0% to 100%, other gas concentration sensors 42 detect the amount of carbon dioxide gas in various curves such a convex curve, a concave curve or a wavy curve. In the case of the gas concentration sensor 42 which does not linearly detect from 0% to 100%, an error is made in the actually used concentration of carbon dioxide in the incubation chamber 4 if the zero point of such a gas concentration sensor 42 is only calibrated. Therefore, in the present invention, a straight line which connects the concentration of the standard gas used in the incubation chamber 4 to the zero point is set to serve as the reference of the gas concentration sensor 42.

Described below is a method of calibrating the gas concentration sensor 42 such that the line which connects the actually used gas concentration used in the incubation chamber 4 to the zero point serves as the reference. In addition, the highly accurate concentration of the carbon dioxide gas hermetically contained in the cylinder 60 is written on the cylinder 60 in which the standard gas is hermetically contained. Therefore, for the reference value of the controller 70, the operator visually recognizes the written concentration, so that the reference value of the concentration of the carbon dioxide gas which is displayed on the display device 32 by the gas concentration measurement unit 30 is corrected on the basis of the cylinder 60.

Then, the controller 70 closes the electromagnetic valves EV1, EV2, EV4 and opens the electromagnetic valve EV3 to operate the air pump 46, so that the standard gas flows into the gas in-take pipe 56 from the cylinder 60 in which the standard gas is hermetically contained. At this moment, the gas circulating from the incubation chamber 4 remains in the pipes 36, 42A and 56. Therefore, a value after a predetermined period of time (about two minutes) is measured, and a plurality of (about three) measurements are performed.

The controller 70 uses the value (concentration) of the standard gas in the cylinder 60 detected by the gas concentration sensor 42 as a new reference value of the actually used gas concentration, and stores a straight line connecting the previously measured and stored zero point with the reference value in the memory as a new reference line of the actually used gas concentration. The controller 70 sets this line as a new reference for the measurement of the gas concentration. The controller 70 performs this calibration, for example, once a day or every few days, and stores the highly accurate gas concentrations ranging from the zero point to concentrations around the actually used gas concentration.

That is, the measurement error made by the temperature, humidity or a change with time is not displayed on the display device 32 as it is. The error is differentially corrected by the controller 70 after measurement, so that the accurate gas concentration in the incubation chamber 4 is displayed on the display device 32. Specifically, when the gas concentration in the incubation chamber 4 detected by the gas concentration sensor 42 is higher (e.g., 2 percent higher) than the concentration of the standard gas in the cylinder 60, the controller 70 subtracts 2 percent from the gas concentration in the incubation chamber 4 detected by the gas concentration sensor 42 and displays the result on the display device 32. When the gas concentration in the incubation chamber 4 detected by the gas concentration sensor 42 is lower (e.g., 2 percent lower) than the concentration of the standard gas in the cylinder 60, the controller 70 adds 2 percent to the gas concentration in the incubation chamber 4 detected by the gas concentration sensor 42 and displays the result on the display device 32.

As a result, even if a measurement error is made in the gas concentration sensor 42, the gas concentration in the incubation chamber 4 can be displayed on the display device 32 with great precision and high accuracy. As there is no connection of the pipes or connection of the standard gas, the controller 70 can automatically perform the zero-point correction using the air taken through the outside air take-in pipe 54 and the correction of the actually used concentration using the standard gas. Thus, the gas concentration sensor 42 can be automatically calibrated in the vicinity of the gas concentration actually used in the incubation chamber 4, and highly accurate calibration can be performed. Although the measurement error of the gas concentration sensor 42 is set at 2%, the measurement error of the gas concentration sensor 42 is not limited to 2% and may be a different value.

The gas in-take pipe 56 branches so that a connector (not shown) is provided in the gas in-take pipe 56 via the electromagnetic valve EV4. A connection hose 62 is connected to the connector. A high-volume cylinder 64 (e.g., a cylinder of about 25 to 30 cm in diameter and about 120 cm in height) is connected to the connection hose 62. The cylinder 64 is provided in, for example, a hospital having a large standard gas facility, and has a great volume of standard gas hermetically contained therein. Thus, in, for example, the hospital having the large standard gas facility, there is no particular need to prepare the cylinder 60 which is smaller than the high-volume cylinder 64 and in which the standard gas is hermetically contained. The existing high-volume cylinder 64 in which the standard gas is hermetically contained can be used. The controller 70 is also calibrated, for example, once a day or every few days. In addition, the high-volume cylinder 64 is not limited to this size.

As described above, the incubation apparatus 1 includes the gas concentration sensor 42 for detecting the gas concentration in the incubation chamber 4, the controller 70 for controlling the supply of the gas into the incubation chamber 4 on the basis of an output of the gas concentration sensor 42, and the standard gas supply means for supplying the gas concentration sensor 42 with a standard gas which has been previously adjusted to the actually used gas concentration or a gas concentration close thereto. The controller 70 maintains the concentration of the standard gas, supplies the standard gas to the gas concentration sensor 42 by the standard gas supply means, and on the basis of an output of the gas concentration sensor 42 and the concentration of the standard gas, calibrates the actually used gas concentration of the gas concentration sensor 42. As a result, the gas concentration sensor 42 can be automatically calibrated at the actually used concentration of the gas in the incubation chamber 4 or a gas concentration close thereto.

Consequently, the gas concentration sensor 42 can be automatically calibrated in the vicinity of the gas concentration actually used in the incubation chamber 4, so that calibration suitable for practical use is performed to enable highly accurate incubation. Especially, there is no need to use, for example, a special gas concentration measurement instrument to measure the gas concentration in the incubation chamber 4, thereby enabling a significant improvement in maintenance work.

Furthermore, the gas concentration measurement unit 30 includes the electromagnetic valve EV2 and the outside air take-in pipe 54 for supplying outside air to the gas concentration sensor 42, and the controller 70 supplies the outside air to the gas concentration sensor 42 by the electromagnetic valves EV2 and the outside air take-in pipe 54, and on the basis of an output of the gas concentration sensor 42, performs zero-point calibration of the gas concentration sensor 42 stored in the controller 70. Thus, the gas concentration sensor 42 can be calibrated at two points including a zero gas concentration and the actually used gas concentration, thereby enabling more accurate calibration.

Still further, the gas concentration measurement unit 30 includes the detachable cylinder 60 in which the standard gas is hermetically contained, and the standard gas is supplied from this cylinder 60 to the gas concentration sensor 42. Thus, there is no need for a pipe for supplying the standard gas from an external gas supply source.

Further yet, the gas concentration measurement unit 30 includes the connector 56A to connect a pipe which supplies the standard gas to the gas concentration sensor 42. Thus, when the incubation apparatus 1 is installed in a facility such as a hospital which is already provided with large standard gas equipment having a line to supply the standard gas, this standard gas line can be connected to the connector 56A via the connection hose 62 to supply the standard gas to the gas concentration sensor 42.

Further yet, in the incubation apparatus 1, the controller 70 calibrates the actually used gas concentration at preset time intervals. This makes it possible to always achieve accurate incubation in the incubation chamber 4, and to hold down the amount of the use of the relatively expensive standard gas.

Although the carbon dioxide gas is used as the incubation gas in the incubation apparatus 1 described in the embodiment, the incubation gas is not limited to the carbon dioxide gas and may be an oxygen gas or a different gas.

It should be understood that the present invention is not exclusively limited to the embodiments described above, and various modifications can be made without departing from the spirit of the invention.

## Claims

1. An incubation apparatus to incubate culture targets such as cells, embryos or microorganisms in an incubation chamber, the incubation apparatus including:
a gas concentration sensor which detects a gas concentration in the incubation chamber;
control means for controlling the supply of a gas into the incubation chamber on the basis of an output of the gas concentration sensor; and
standard gas supply means for supplying the gas concentration sensor with a standard gas which has been previously adjusted to an actually used gas concentration or a gas concentration close thereto,
wherein the control means maintains the concentration of the standard gas, supplies the standard gas to the gas concentration sensor by the standard gas supply means, and on the basis of an output of the gas concentration sensor and the concentration of the standard gas at the moment, calibrates the actually used gas concentration of the gas concentration sensor.

2. The incubation apparatus according to claim 1, further including
outside air supply means for supplying outside air to the gas concentration sensor,
wherein the control means supplies the outside air to the gas concentration sensor by the outside air supply means, and on the basis of an output of the gas concentration sensor at the moment, performs zero-point calibration of the gas concentration sensor.

3. The incubation apparatus according to claim 1 or 2, further including a detachable cylinder in which the standard gas is hermetically contained, the standard gas being supplied from the cylinder to the gas concentration sensor.

4. The incubation apparatus according to any one of claims 1 to 3, further including a connection opening to connect a pipe which supplies the standard gas to the gas concentration sensor.

5. The incubation apparatus according to any one of claims 1 to 4, wherein the control means calibrates the actually used gas concentration at preset time intervals.
